# EUROPÄISCHE PATENTANMELDUNG

(11) **EP 2 018 878 A1**
(43) Veröffentlichungstag der Anmeldung: **28.01.2009**
(21) Anmeldenummer: 07014516.4
(22) Anmeldetag: 25.07.2007
(51) Int. Cl.: A61L 27/04, A61L 27/10, A61L 27/30, A61C 8/00

(54) **Keramikimplantate- Zirkonimplantate- mit einer Titan- oder Titanoxidbeschichtung des intraossären Teiles**

(71) Anmelder: Lenz, Sorin, Dr., 5020 Salzburg (AT)
(72) Erfinder: Lenz, Sorin, Dr., 5020 Salzburg (AT)
(74) Vertreter: Vossius & Partner

(57) **Zusammenfassung**

Implantate für die Medizin, Zahnmedizin, Tiermedizin Fig.1, mit einem Implantatkörper (Grundkörper) aus Keramik -Zirkon- 1, und mit einer Oberflächenbeschichtung aus Titan, Titanoxid oder einer anderen biokompatiblen Titanverbindung beschichtet. Mit Titan, Titanoxid oder einer anderen biokompatiblen Titanverbindung, werden diejenigen Bereiche des Implantates beschichtet 3, welche sich nach der Implantation im Knochen befinden (intraossär) 5, um dort besser knöchern einheilen zu können (bekannt gute Osseointegration des Titans). Die restlichen Bereiche des Implantates welche nach dem Implantieren nicht im Knochen eingebracht sind 2 bleiben unbeschichtet, damit keine unerwünschten Anlagerungen stattfinden, und die positiven Eigenschaften der Keramik -Zirkon- voll zur Geltung kommen. Die nicht nur ästhetischen Vorteile in der Zahnmedizin sollten keineswegs außer Acht gelassen werden, denn dadurch wurde erst die Erfindung zum Wohl der Patienten möglich, nämlich die erfindungsgemäße Kombination zweier bekannter Werkstoffe.

## Beschreibung

Die Erfindung bezieht sich auf das Gebiet der Medizin und Zahnmedizin, sowie der Tiermedizin, im speziellen das Gebiet der Implantologie.

Die Erfindung bezieht sich auf Implantate, mit einem Implantatkörper (Grundkörper) aus Keramik -Zirkon-, und mit einer Oberflächenbeschichtung aus Titan, oder Titanoxid. Mit Titan oder Titanoxid werden diejenigen Bereiche des Keramikkörpers - Zirkon - beschichtet, welche sich nach der Implantation in Knochenkontakt befinden, um dort besser knöchern einheilen zu können (Osseointegration). Die restlichen Bereiche des Implantates, welche nach der Implantation nicht mit dem Knochen Kontakt haben, bleiben unbeschichtet.

Im allgemeinen dienen Implantate als Ersatz für erkrankte oder verloren gegangene menschliche oder tierische anatomische Strukturen, wie: Zähne, Gelenke, Extremitäten usw. Vorzugsweise sollten solche Implantate mit dem Knochen im Organismus verwachsen um eine stabile, belastbare Verbindung zu bilden. Es gibt bereits sowohl Titanimplantate, als auch Keramikimplantate. Während Titanimplantate ihren festen Platz in der Medizin, Zahnmedizin und Tiermedizin mit über 30 jähriger Erfahrung eingenommen haben, werden Keramikimplantate erst seit kurzer Zeit in der Implantologie verwendet.

Ziel der Erfindung ist die sichere Osseointegration keramischer - Zirkon- Implantate sowie die Nutzung der herausragenden Ästhetik.

Die Erfindung beruht auf die bestimmte Kombination zweier bekannter, biokompatibler Materialien für Implantate, welche **nicht** so kombiniert, einzeln, bereits in der Medizin verwendet werden.

Durch die Erfindung werden zwei grundlegende Probleme der Zahnmedizin gelöst: Die geforderte optimale Ästhetik bei optimaler Osseointegration.

Für die Medizin und Tiermedizin steht die sichere Osseointegration der mit Titan, Titanoxid beschichteten Keramik -Zirkon- Implantate im Vordergrund.
Beide oben erwähnte Materialien, Titan und Keramik -Zirkon-, sind in der Medizin bekannt, ihre Vor- und Nachteile ebenso. Die Erfindung kombiniert und bündelt die Vorteile beider Materialien optimal zum Wohle der Patienten, während die Nachteile minimiert, wenn nicht sogar eliminiert werden.

Doch zuerst zu den beiden Materialien, und ihre Eigenschaften im einzelnen:

Keramik -Zirkon- ist ein extrem hartes, glattes und biologisch inertes Material, welches gegen Korrosion (Säure, Salze, Körperflüssigkeiten) absolut beständig ist. Des Weiteren ist es wegen seiner Härte extrem abriebfest - das heißt die Oberfläche kann nur mit Diamantwerkzeug verändert werden.
Weiterhin sollte nicht unerwähnt bleiben, dass die weiße Farbe des Materials zumindest für Zahnimplantate in der Zahnmedizin herausragende ästhetische Vorteile bietet.
Diese sehr wünschenswerten Eigenschaften, welche dieses Material besitzt werden bereits in der Medizin genutzt, z.B. als Stents für Gefäße in der Kardiologie, mit einer Oberfläche dieser Stents aus Keramik damit keine Anlagerungen von Körperzellen (Blutbestandteile) stattfindet. In der Zahnmedizin gibt es Zahnimplantate aus Keramik -Zirkon- seit nicht allzu langer Zeit, welche verloren gegangene Zähne ersetzen sollen. Die weiße Farbe ist ästhetisch sehr wünschenswert, aber in diesem Fall sind die oben erwähnten Vorteile ein Nachteil, denn dadurch dass das Material so biologisch inert ist, wird das Implantat im

Knochen nicht (oder nur ungenügend) osseointegriert. (Die Experten diskutieren darüber kontrovers ob Keramik -Zirkon- überhaupt osseointegriert, da es noch keine gesicherten Langzeitergebnisse darüber gibt.)

Titan, ein seit über 30 Jahren bekanntes Material in der Implantologie, hat die Vorteile, dass es extrem gut osseointegriert (mit dem Knochen verwächst), und nicht allergen ist. Knochen verwächst im Sinne einer Ankylose (ähnlich einem geheilten Knochenbruch) mit der obersten Titanschicht, weshalb heutige Titanimplantate eine Oberflächenanrauung erfahren, um die Kontaktfläche zwischen Implantat und Knochen, so weit wie technisch möglich zu maximieren (Oberfächenvergrößerung). Damit wird die Osseointegration noch weiter verbessert.
Titan hat folgende, speziell für die zahnärztliche Implantologie eminenten Nachteile: Es hat eine dunkle, fast schwarze Farbe, und falls es hochglanzpoliert ist eine silbrige Farbe, wodurch die Ästhetik im Zahnhalsbereich sehr zu wünschen übrig lässt. (Deshalb wurden, zur Verbesserung der Ästhetik, wie oben bereits erwähnt, vor nicht allzu langer Zeit weiße Keramikimplantate entwickelt).
Des weiteren, lassen sich Titanimplantate in der Zahnmedizin an der Durchtrittsstelle aus dem Zahnfleisch nicht mit Ultraschallspitzen aus Metall reinigen, da das Material dadurch zerkratzt wird, und damit Rauhigkeiten entstehen, welche vermehrte Zahnbelagbildung fördern. (Zur Reinigung werden spezielle Kunststoffspitzen benötigt).

Ziel der Erfindung ist es, durch die erfindungsgemäße Kombination beider beschriebenen Materialien, (beide am Markt erhältlich), die maximal möglichen Vorteile beider Werkstoffe zu nutzen, und genau dort wo es gewünscht ist, die Nachteile der einzelnen Materialien zu eliminieren.
Im folgenden wird die Erfindung anhand zweier Beispiele aus der Zahnmedizin, Medizin und Tiermedizin und mit Bezug auf die beigefügten Zeichnungen veranschaulicht, in denen,
Fig. 1 Zahnmedizin, Kieferorthopädie und Kieferchirurgie (Mensch, Tier), eine schematisch Zeichnung eines im Kieferknochen inseriertes Zahnimplantates darstellt, ohne Suprakonstruktion (ohne Zahnkrone), und
Fig. 2 Medizin, Tiermedizin eine schematisch Zeichnung eines im Oberschenkel- und Beckenknochen eingesetztes Hüftgelenksimplantates darstellt.

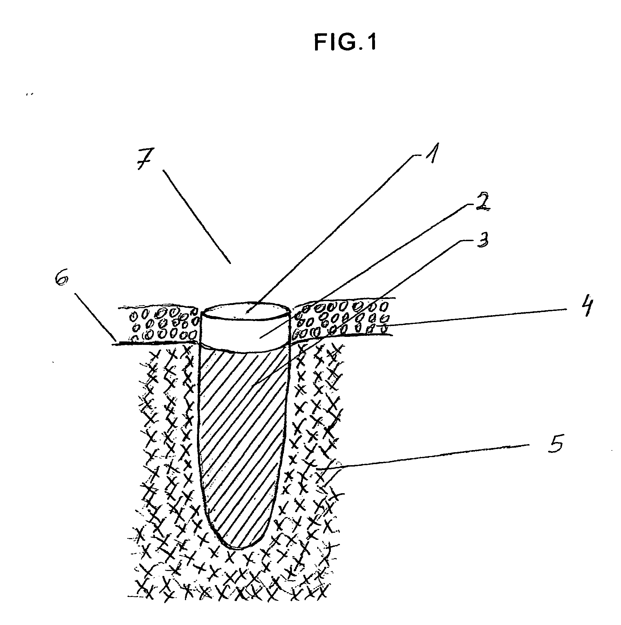

In der Zahnmedizin Fig.1 ist eine weiße Farbe im Bereich oberhalb der Knochenkante 6 bis zur Durchtrittsstelle des Implantates durch das Zahnfleisch (Weichgewebe) 4 in den Mundraum 7, von größter ästhetischer Wichtigkeit, denn Zähne sind bekannter Maßen weiß. Der weiße Keramikkörper -Zirkon- 1 erfüllt diesen ästhetischen Anspruch optimal und zur Gänze, speziell im nicht beschichteten supraossären Anteil 2. Des Weiteren lässt sich die Keramik -Zirkon- Oberfläche 2, nicht mit Metall Ultraschallspitzen verletzen (zerkratzen),da sie extrem glatt und hart ist, was eine geringere oder keine Anlagerung von Zahnbelag zur Folge hat. Die Keramik wird auch durch die Flüssigkeiten im Mund nicht angegriffen 2, es ist biologisch inert und Korrosionsbeständig.
Die vollständige Titan- oder Titanoxidbeschichtung 3 des Keramik - Zirkon- Implantatkörpers 1 im intraossären Bereich 2 ermöglicht die gewünschte und bereits von Titanimplantaten bekannte, optimale Osseointegration des Implantates. Damit wächst das Implantat im umgebenden Knochen 5 sehr gut ein. Die gute Osseointegration des Titans ist eine seit vielen Jahrzehnten bekannte, erforschte und bestätigte, in der Medizin genutzte Eigenschaft dieses Werkstoffes. Durch die Titan, Titanoxidbeschichtung 3 der Keramik -Zirkon-1 Implantate erreicht man die Kombination aus optimaler Ästhetik 2 und optimaler Osseointegration 3,5 (nach heutigem Wissensstand).

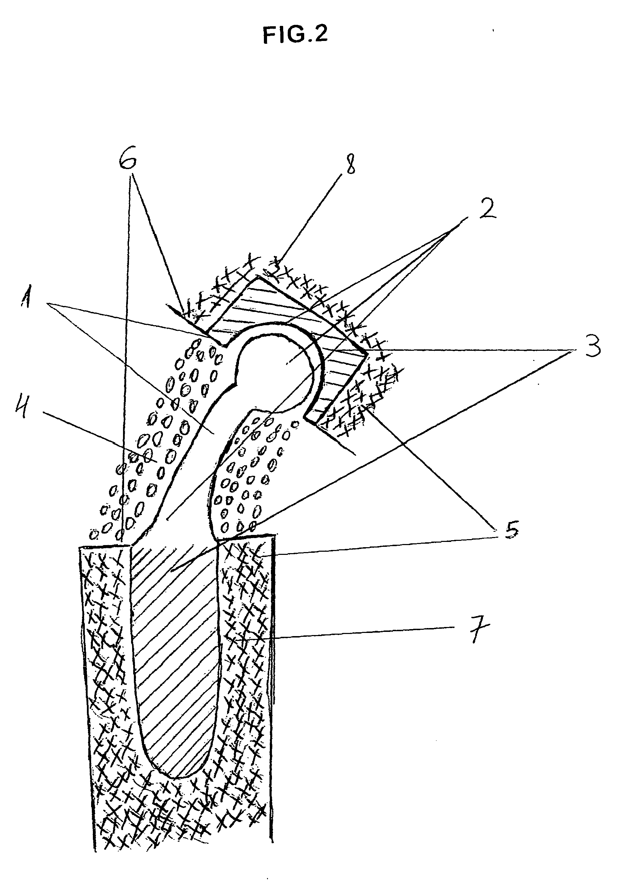

In der Medizin oder Tiermedizin Fig. 2 (Orthopädische Chirurgie z. B), bei dem Ersatz erkrankter nicht mehr heilbarer Gelenke, wie z.B. Hüftgelenke Fig. 2, (auch bei Kniegelenke, Fingergelenke usw.), hat die Ästhetik im Gegensatz zur Zahnmedizin, zwar keine besondere Bedeutung, aber die
Materialeigenschaften und die erfindungsgemäße Kombination beider Werkstoffe, ermöglichen durch die vollständige Titan- 3 Titanoxid Beschichtung des intraossären Anteils 3 des Keramik -Zirkon-Implantatkörpers optimale, wissenschaftlich seit Jahrzehnten bestätigte Osseointegration. Die vollständig Titan beschichteten Anteile 3 des Hüftgelenksimplantates 1, befinden sich im Oberschenkelknochen 7 und im Beckenknochen 8.
Die Anteile 2 welche sich im Weichgewebe 4 befinden, und die Gelenkflächen (Hals, Kopf und Pfanne) 2 bleiben unbeschichtet, da Keramik -Zirkon- wie bereits erwähnt biologisch und chemisch inert ist. Damit finden keine Anlagerungen statt, und keine
Fremdkörperreaktionen.
Die außergewöhnliche Härte sowie die Glätte der Keramik -Zirkonprädestinieren den Werkstoff zur Gelenkflächenherstellung 2, da mechanische Abnutzung, z.B. durch Abrieb, absolut unerwünscht wäre und durch die Materialeigenschaften bedingt, auch nicht stattfindet.

Bei Versuchen wurde festgestellt, dass beim erfindungsgemäßen Implantat die Beschichtung mit Titan- Titanoxid sich nur mit rotierenden Instrumenten entfernen lässt. Zahnärztliche Ultraschall Instrumente betrieben mit 100% der Leistung (Maximalleistung) mit Metallspitze, konnten die Beschichtung weder entfernen, noch abplatzen lassen. Auch das Einspannen in einem großen Schraubstock mit 35 cm. Hebel ließ die Beschichtung nicht abplatzen, trotz Zudrehen mit der maximalen Kraft eines Erwachsenen. Lediglich die Abdrücke der Backen waren sichtbar. Die Beschichtung blieb intakt, und platzte auch nicht ab.
Die Titanschichtdicke betrug bei den Versuchen 6-16µm, es sind aber selbstverständlich Schichtdicken von 0,5 bis 50µm und darüber mit geeigneten Beschichtungsverfahren möglich.

Selbstverständlich kann die Titan, Titanoxid Beschichtung nachdem sie vollständig den intraossären Teil des Implantates bedeckt, auch als Titanhülse der Keramik -Zirkon- Implantate verstanden werden. Teilbeschichtete (jede Teilmenge eines Ganzen) intraossäre Anteile des Keramik -Zirkon- Implantates bis zur Knochenaustrittsstelle, in Form von Titan, Titanoxid Ringen, Streifen (vertikal, horizontal, diagonal), Punkten oder Flecken würden sicherlich auch zu einer besseren Osseointegration führen als komplett Titan Titanoxid **unbeschichtete** Keramik- Zirkon- Implantate.

Die erfindungsgemäßen Implantate finden selbstverständlich auch in der Kieferorthopädie und Kieferchirurgie Anwendung.
Für alle erfindungsgemäßen Anwendungsbereiche gilt, dass alle geometrischen, und nicht geometrischen Implantatformen zur Anwendung kommen können.
Jedes erfindungsgemäße Implantat mit jeder Form kann selbstverständlich mit jeder Art eines Gewindes nach dem Stand der Technik versehen werden, und die Oberfläche welcher Form auch immer, mit oder ohne Gewinde, kann im intraossären Anteil angeraut sein zur Oberflächenvergrößerung. Die Anrauung kann vor oder auch nach der Beschichtung erfolgen nach dem Stand und den Methoden der Technik.
Selbstverständlich kann jedes erfindungsgemäße Implantat wie oben beschrieben (jede Form, mit und ohne Gewinde) auch eine glatte Oberfläche haben, sogar eine hochglanzpolierte, auch wenn dies weniger sinnvoll ist als Oberflächenanrauung des intraossären Bereiches.

## Patentansprüche

1. Keramikimplantate -Zirkonimplantate-, Gesamtimplantatkörper (Grundkörper) aus Keramik -Zirkon- (1), mit Titan oder Titanoxid beschichtet, oder beschichtet mit einer anderen biokompatiblen Titanverbindung, **gekennzeichnet durch** eine sich auf die intraossär zu inserierenden Bereiche des Implantates erstreckende vollständige Oberflächenbeschichtung aus Titan, Titanoxid (3) oder eine andere biokompatible Titanverbindung. Die restlichen Bereiche des Implantates, (Grundkörpers) extraossär bleiben unbeschichtet (2).

2. Keramikimplantate -Zirkonimplantate- nach Anspruch 1, wobei alle geometrischen und nicht geometrischen Implantatformen Anwendung finden, und diese auch mit einem Gewinde nach dem Stand der Technik versehen sein können, aber nicht sein müssen.

3. Keramikimplantate - Zirkonimplantate-, nach Anspruch 1 und 2, wobei der intraossäre Bereich sowohl oberflächenangeraut als auch glatt oder sogar hochglanzpoliert sein kann

4. Keramikimplantate - Zirkonimplantate-, nach Anspruch 1, 2 und 3, wobei die vollständige Beschichtung intraossär (3) auch als Hülse verstanden werden kann.

5. Keramikimplantate - Zirkonimplantate- nach Anspruch 1, 2 und 3, wobei die intraossäre Beschichtung auch nur partiell sein kann, das heißt jede Teilmenge kann verwendet werden, welche noch Osseointegration gewährleistet.

6. Keramikimplantate -Zirkonimplantate-, mit Titan oder Titanoxid Beschichtung, oder Beschichtung mit einer anderen biokompatiblen Titanverbindung, nach Anspruch 1 und 2, wobei die Beschichtung den gesamten Implantatkörper umschließt.
